# EUROPÄISCHE PATENTANMELDUNG

(11) **EP 0 853 123 A1**
(43) Veröffentlichungstag der Anmeldung: **15.07.1998**
(21) Anmeldenummer: 97100331.4
(22) Anmeldetag: 10.01.1997
(51) Int. Cl.: C12N 15/10, C07H 1/08, B01D 61/00

(54) **Reinigung von DNA durch Cross-Flow-Filtration**

(71) Anmelder: BOEHRINGER MANNHEIM GMBH, 68305 Mannheim (DE)
(72) Erfinder: Die Erfindernennung liegt noch nicht vor
(74) Vertreter: Weiss, Wolfgang, Dipl.-Chem. Dr.

(57) **Zusammenfassung**

Die Erfindung betrifft ein Verfahren zur Reinigung oder/und zur Konzentrierung von Nucleinsäuren in einer Lösung, wobei man die Nucleinsäure enthaltende Lösung tangential an einer oder mehreren semipermeablen Membranen vorbeileitet, so daß die Nucleinsäure-Moleküle von den Membranen zurückgehalten werden und Substanzen mit einem geringeren Molekulargewicht durch die Membranen durchtreten können oder/und an der Membran adsorbiert werden und man eine gereinigte oder/und konzentrierte Nucleinsäure-Lösung erhält. In derselben Weise wird zur Abtrennung von Endotoxinen aus einer Nucleinsäure-Präparation verfahren. Weiter betrifft die Erfindung die Verwendung einer Cross-Flow-Filtrationsanlage zur Reinigung oder/und zur Konzentrierung von Nucleinsäuren in einer Lösung sowie zur Abtrennung von Endotoxinen aus einer Nucleinsäure-Präparation. Ferner die Verwendung der mit der Cross-Flow-Filtration gereinigten oder/und konzentrierten Nucleinsäuren zum Klonieren, zur Transformation, zur Transfektion, zur Mikroinjektion in Zellen, zur Verwendung bei Verfahren der Gentherapie oder/und zur Polymerase-Ketten-Reaktion (PCR).

## Beschreibung

Die vorliegende Erfindung betrifft ein Verfahren zur Reinigung oder/und zur Konzentrierung von Nucleinsäuren in einer Lösung, ein Verfahren zur Abtrennung von Endotoxinen aus einer Nucleinsäure-Präparation sowie die Verwendung einer Cross-Flow-Filtrationsanlage zur Reinigung oder/und zur Konzentrierung von Nucleinsäuren in einer Lösung und zur Abtrennung von Endotoxinen aus einer Nucleinsäure-Präparation.

Im Bereich der Molekularbiologie sind Nucleinsäure-Aufreinigungsverfahren gebräuchliche Methoden. In aus dem Stand der Technik bekannten Verfahren wird z. B. das gewonnene biologische Material, beispielsweise E.coli Bakterienzellen, nach dessen Aufschluß (üblicherweise Lyse mit Lysozym oder Ultraschall) abzentrifugiert und der Überstand wird mit Phenol ausgeschüttelt. Anschließend wird an einem Cäsiumchlorid-Gradienten eine Ultrazentrifugation durchgeführt (Birnboim & Doly, Nucl. Acid Res. 7 (1979) 1513 - 1523, Garger et al., Biochem. Biophys. Res. Comm. 117 (1983) 835-842). Derartige Präparationen enthalten jedoch im allgemeinen als Verunreinigungen bakterielle Endotoxine, Phenol, Cäsiumchlorid und/oder Ethidiumbromid.

Endotoxine sind Bakterientoxine, die bei allen Enterobacteriaceae, z. B. Salmonella, Shigella und E.coli zu finden sind. In Säugern wirken Endotoxine als Pyrogen und rufen neben Fieber zahlreiche weitere pathologische Wirkungen hervor. Für die toxische Wirkung der Endotoxine ist insbesondere der Bestandteil Lipid A verantwortlich.

Ein anderes Verfahren zur Aufreinigung von Nucleinsäuren ist im QIAGEN® Plasmid-Handbook (Qiagen Inc., Chatsworth, USA) und der EP-B 0 268 946 beschrieben. Danach wird das nach üblichem Aufschluß gewonnene Zell-Lysat an QIAGEN®-TIP, welches QIAGEN® resin (ein Trägermateral auf Silicagelbasis) enthält, chromatographiert. Der Nachteil dieses Verfahrens ist, daß DNA-Bindeproteine nicht vollständig von der DNA abgelöst werden, so daß die erhaltene Plasmid-Präparation Proteine und insbesondere Endotoxine (z. B. aus der Membran der E.coli-Zelle) in beträchtlichem Umfang enthält.

In einem weiteren Nucleinsäure-Aufreinigungsverfahren wird nach alkalischer Lyse des biologischen Materials, beispielsweise E.coli-Zellen, nach Birnboim & Doly eine Chromatographie des Zentrifugationsüberstandes, unter Hochsalzbedingungen über Anionentauschersäulen (z. B. Mono-Q, Source-Q von Pharmacia, Macroprep-Q von Biorad, Poros-Q von Perspektive Biosystems oder HyperD-Q von Biosepra, vgl. Chandra et al., Analyt. Biochem. 203 (1992) 169-172; Dion et al., J. Chrom. 535 (1990) 127-147) durchgeführt. Auch nach diesem Reinigungsschritt enthält die Plasmid-Präparation noch Verunreinigungen wie Proteine und insbesondere in beträchtlichem Umfang Endotoxine.

In noch einem weiteren Verfahren zur Aufreinigung von Nucleinsäuren wird nach alkalischer Lyse und anschließender Phenol-Chloroform-Extraktion eine Chromatographie über Gelfiltration durchgeführt (McClung & Gonzales, Analyt. Biochem. 177 (1989) 378-382; Raymond et al., Analyt. Biochem. 173 (1988) 124-133). Auch dieses Reinigungsverfahren kann die Verunreinigungen nicht vollständig aus der Plasmid-Präparation entfernen.

Den genannten Aufreinigungsmethoden ist ein abschließender Entsalzungs- und Konzentrierungsschritt gemeinsam. Üblicherweise wird dabei eine Isopropanol/Ethanol-Fällung der Nucleinsäure mit anschießender Zentrifugation und Resuspension des Nucleinsäure Pellets in Puffer angewendet (vgl. z. B. Sambrook J. et al. (1989), Molecular Cloning; A Laboratory Manual, 2nd Ed., Cold Spring Harbor Laboratory Press). Hierbei wird beispielsweise eine DNA-Lösung mit 1/10 Volumen 4 M LiCl und anschließend mit 0,7 Volumen Isopropanol bei Raumtemparatur versetzt. Anschließend wird der sich bildende Niederschlag der Nucleinsäure abzentrifugiert und der Überstand verworfen. Das Pellet, das die Nucleinsäure enthält, wird in einem folgenden Schritt in 70%igem Ethanol aufgenommen, nochmals zentrifugiert, der Überstand wird verworfen und nach Trocknung des Pellets wird dieses in einem gewünschten Puffer resuspendiert. Die Isopropanol/Ethanol-Fällungsmethode ist jedoch nur für Anwendungen im Labor praktikabel, wo mit relativ kleinen Volumina gearbeitet wird.

Abgesehen von der Beschränkung auf kleine Volumina hat die beschriebene Isopropanol/Ethanol-Fällungsmethode noch weitere erhebliche Nachteile. So ist es aus Gründen der Betriebssicherheit und des Umweltschutzes sehr ungünstig, die im industriellen Prozeßmaßstab nötigen Isopropanol/Ethanol-Volumina zu verwenden. Außerdem erfüllt die Isopropanol/Ethanol-Fällungsmethode nicht die technischen Voraussetzungen, um therapeutisch verwendbare Nukleinsäuren bereitzustellen, da in der Nucleinsäure-Lösung enthaltene Endotoxine mit dieser Methode nicht vollständig entfernt werden können.

In der WO 95/21177 wird ein Verfahren zur Isolierung und Reinigung von Nucleinsäuren für den Einsatz in der Gentherapie beschrieben, wobei die Reinigung im wesentlichen durch Zentrifugieren, Filtrieren, eine Affinitätschromatographie oder eine Chromatographie an einem anorganischen Chromatographiematerial und eine Chromatographie an einem Ionenaustauscher erfolgt. Zur Abreicherung von Endotoxinen wird die Nucleinsäure mit einem "Endotoxin Removal Buffer", der 10 % Triton® X 100 und 40 mmol/l MOPS-Puffer (3-Morpholino-1-Propansulfonat) enthält, behandelt. Ein Nachteil dieses Verfahrens besteht darin, daß die auf diese Weise gereinigte Nucleinsäure Verunreinigungen der pharmakologisch nicht unbedenklichen Substanzen Triton® und MOPS enthält. Außerdem kann zwar eine Abreicherung von Endotoxinen auf einen Gehalt von ca. 100 EU/mg DNA erreicht werden (QIAGEN News 1-96, 3-5), eine weitergehende Entfernung von Endotoxinen ist jedoch nicht möglich. Für eine therapeutische Anwendung, wie sie beispielsweise für die Gentherapie vorgesehen ist, werden jedoch Nucleinsäure-Präparationen mit noch höherer Reinheit benötigt, die möglichst frei von allen Verunreingungen (insbesondere weitestgehend frei von Endotoxinen) sind. Vor allem der Endotoxingehalt von Plasmid-DNA-Präparationen stellt bisher ein nicht gelöstes Problem dar, wie beispielsweise von Cotten et al., Gene Therapy 1 (1994) 239 - 246 beschrieben wurde.

In EP-A 96 101 628.4 wird vorgeschlagen, Nucleinsäure-Präparationen über Anionenaustauscher mittels eines Hochsalzgradienten zu reinigen, um Nucleinsäure-Lösungen zu erhalten, die einen Proteingehalt von weniger als 0,1 % haben, frei von Verunreinigungen, wie etwa Ethidiumbromid, Phenol, Cäsiumchlord und Detergenzien sind und die weitgehend frei von Endotoxinen sind.

Aus dem Stand der Technik sind somit weder Verfahren bekannt, die es ermöglichen große Nucleinsäuremengen zu reinigen oder zu konzentrieren, noch sind Verfahren bekannt, mit denen Nucleinsäuren in großen Mengen und in hochreiner Form, insbesondere frei von Endotoxinen, die für eine therapeuthische Verwendung geeignet sind, bereitzustellen.

Eine der vorliegenden Erfindung zugrundeliegende Aufgabe war es daher, ein Reinigungs- und Konzentrationsverfahren bereitzustellen, das es auf einfache Weise ermöglicht, Nucleinsäuren in großen Mengen aufzureinigen. Eine weitere der vorliegenden Erfindung zugrundeliegende Aufgabe bestand darin, die Konzentration an Endotoxinen in einer Nucleinsäure-Präparation soweit zu reduzieren, daß sie für den therapeutischen Einsatz geeignet ist.

In einem ersten Aspekt betrifft die vorliegende Erfindung ein Verfahren zur Reinigung oder/und zur Konzentrierung von Nucleinsäuren in einer Lösung, welches dadurch gekennzeichnet ist, daß die Nucleinsäure enthaltende Lösung tangential an einer oder mehreren semipermeablen Membranen vorbeigeleitet wird, so daß die Nucleinsäure-Moleküle von den Membranen zurückgehalten werden und Substanzen mit einem geringeren Molekulargewicht durch die Membranen durchtreten können, wobei eine gereinigte oder/und konzentrierte Nucleinsäure-Lösung erhalten wird.

In einem weiteren Aspekt betrifft die vorliegende Erfindung ein Verfahren zur Abtrennung von Endotoxinen aus einer Nucleinsäure-Präparation, welches dadurch gekennzeichnet ist, daß die Nucleinsäure enthaltende Präparation tangential an einer oder mehreren semipermeablen Membranen vorbeigeleitet wird, so daß die Nucleinsäure-Moleküle von den Membranen zurückgehalten werden und Substanzen mit einem geringeren Molekulargewicht durch die Membranen durchtreten können oder/und von der Membran adsorbiert werden, wobei eine im wesentlichen endotoxinfreie Nucleinsäure-Lösung erhalten wird.

Es wurde nun festgestellt, daß sich mit dem Verfahren der vorliegenden Erfindung unter Verwendung einer Cross-Flow-Filtrationsanlage Nucleinsäure-Lösungen reinigen und konzentrieren lassen. Überraschend und neu ist dabei insbesondere, daß die Nucleinsäuren durch die Cross-Flow-Filtration (CFF) nicht geschädigt werden. Man ging bisher immer davon aus, daß die bei der CFF auftretenden Scherkräfte zu Schädigungen von Nucleinsäuren, insbesondere zu Strangbrüchen führen würden. Deshalb wurde die CFF bisher nur zur Konzentrierung und Diafiltration von Proteinen eingesetzt. Außerdem können mit dem Verfahren der Erfindung nicht nur Nucleinsäuren in großen Mengen und gewünschter Reinheit erhalten werden, sondern das Verfahren der vorliegenden Erfindung vermeidet auch die Verwendung von organischen Lösungsmitteln, was in toxikologischer Hinsicht sowie unter Sicherheits- und Umweltaspekten von großem Vorteil ist.

Überraschenderweise wurde darüber hinaus festgestellt, daß mit dem Verfahren der vorliegenden Erfindung auch Nucleinsäure-Präparationen gewonnen werden können, die weitgehend von Verunreinigungen, insbesondere von Endotoxinen, befreit sind, so daß sie in therapeutischen Verfahren oder Anwendungen eingesetzt werden können. Damit löst das vorliegende Verfahren ein Problem, das durch den stark steigenden Mengenbedarf an therapeutisch einsetzbaren Nucleinsäuren, insbesondere an therapeutisch einsetzbarer DNA entstanden ist und erwartungsgemäß in Zukunft weiter ansteigen wird.

Mit dem Verfahren der vorliegenden Erfindung werden lineare oder zirkuläre Nucleinsäuren, vorzugsweise Plasmid-DNA und am meisten bevorzugt zirkuläre Plasmid-DNA gereinigt oder/und konzentriert. Die Größe der Nucleinsäure liegt dabei vorzugsweise im Bereich von ≥ 150 Basenpaaren, besonders bevorzugt im Bereich von 1 kbp - 200 kbp. Die Nucleinsäure kann in dem erfindungsgemäßen Verfahren chargenweise gereinigt oder/und konzentriert werden, vorzugsweise wird das Verfahren kontinuierlich durchgeführt. Jede Volumengröße kann prozessiert werden, wobei vorzugsweise eine Lösung mit einem Volumen von 1 bis 10.000 l, besonders bevorzugt mit 1 - 100 l aufgearbeitet wird. Die die Nucleinsäuren enthaltende Lösung wird unter geeigneten Druckbedingungen an der oder den Membranen vorbeigeleitet, wobei der Überströmdruck vorzugsweise größer als der Transmembrandruck ist. Besonders bevorzugt wird unter einem Transmembrandruck von 0,2 bis 3,0 bar, am meisten bevorzugt von 0,8 - 1,5 bar gearbeitet, wobei der Überströmdruck größer als der Transmembrandruck ist. Die Retentatfließrate (RF) kann über einen weiten Bereich variiert werden, vorzugsweise wird mit einer RF von 100 l/h·m² bis 4.000 l/h·m² gearbeitet. Das Verfahren kann auch bei variierenden Temperaturen durchgeführt werden, vorzugsweise wird in einem Temperaturbereich von 4° C - 25° C gearbeitet.

Um die Nucleinsäuren enthaltende Lösung von niedermolekularen Verunreinigungen und insbesondere von Endotoxinen zu trennen, werden gebräuchliche Membranen, wie etwa Membranen aus Polyethersulfon (PES) modifiziertem PES, Polyvinylidendifluorid (PVDF), Cellulosetriacetat oder regenerierter Cellulose verwendet. Ebenfalls für das erfindungsgemäße Verfahren geeignet sind Hohlfaserwickelmodule. Vorzugsweise werden Membranen mit einer Ausschlußgröße von 1 - 1000 Kilodalton (kD), mehr bevorzugt von 10 - 300 kD und am meisten bevorzugt von 10 - 100 kD, verwendet. Der Endotoxinabreicherungsfaktor (Endotoxingehalt der Nucleinsäure-Präparation vor Cross-Flow-Filtration zu Endotoxingehalt der Nucleinsäure-Lösung nach Cross-Flow-Filtration), der in dem vorliegenden Verfahren erreicht wird, beträgt dabei mindestens 10 : 1, vorzugsweise mindestens 200 : 1. Der Endotoxingehalt der Lösung ist nach der Cross-Flow-Filtration sehr gering, er beträgt vorzugsweise < 0,1 EU/mg Nucleinsäure. Die in dem vorliegenden Verfahren erhaltenen Nucleinsäuren sind im wesentlichen unbeschädigt und weisen im wesentlichen keine Einzel- oder Doppelstrangbrüche auf.

Insbesondere zeigt eine erfindungsgemäß gereinigte Plasmid-DNA nach gelektrophoretischer Auftrennung nur eine dominante Bande, die der Konformation "Covalently Closed Circle" entspricht. Des weiteren sind neben geringen Anteilen der Konformationen "Open Circle" und "Linearized Circle" keine weiteren Banden vorhanden.

In einem weiteren Aspekt betrifft die vorliegende Erfindung die Verwendung einer Cross-Flow-Filtrationsanlage zur Reinigung oder/und zur Konzentrierung von Nucleinsäuren in einer Lösung.

In noch einem weiteren Aspekt betrifft die vorliegende Erfindung die Verwendung einer Cross-Flow-Filtrationsanlage zur Abtrennung von Endotoxinen aus einer Nucleinsäure-Präparation.

In noch einem weiteren Aspekt betrifft die vorhergehende Erfindung die Verwendung der mit der Cross-Flow-Filtration gereinigten oder/und konzentrierten Nucleinsäuren zum Klonieren, zur Transformation, zur Transfektion, zur Mikroinjektion in Zellen, zur Verwendung bei Verfahren der Gentherapie oder/und zur Polymerase-Ketten-Reaktion (PCR).

### Beispiele

In den beschriebenen Versuchen wurden Membranen des Typs "OME-GA" aus modifiziertem Polyethersulfon der Firma Filtron (Best-Nr. δS 100CO1 Ausschlußgrenze 100 kD), Membranen aus Celluloseacetat der Firma Sartorius oder PVDF Membranen der Firma Millipore verwendet. Insbesondere zur Endotoxinabtrennung wurde eine Membran mit der Ausschlußgrenze von 100 Kilodalton verwendet. Zur Überprüfung der Abtrennung von Endotoxinen wurden als Aufstocklösung E-Toxate® der Firma Sigma (Bestell-Nr.: 210) verwendet. Die Prüfung auf Endotoxine erfolgte nach der "Festgel-Methode", in der die Zugabe einer endotoxinhaltigen Lösung zu einer Limulus-Amöbozyten-Lysat-Lösung (LAL-Lösung) eine Gelbildung des Gemisches verursacht. Der Gelbildung liegt eine in mehreren Schritten ablaufende Gerinnungskaskade zugrunde.

### 1. Cross-Flow-Filtration (CFF) einer Plasmid-DNA-Lösung

Zur Überprüfung der CFF als Methode zur Konzentrierung von Plasmid-DNA wird ein Produktionsansatz von 2.000 g E.coli-Biomasse durch eine Alkali-Lyse aufgeschlossen, über Q-Sepharose und Hydroxylapatit-Chromatographie prozessiert, und die erhaltene Plasmid-DNA-Lösung als Ausgangslösung in die CFF eingesetzt.

### 1.1. Herstellung einer Ausgangslösung

2.000 g feuchte E.coli Biomasse wird aus dem Fermenter in entpyrogenisierte Zentrifugenbecher abgefüllt. Es werden 22,5 l Resuspensionspuffer (50 mmol/l Tris-HCl, 10 mmol/l EDTA-Na₂, ph 8 ± 0,2) zugegeben und mindestens 24 Stunden bei 5 ± 4° C langsam (ca. 35 RPM) gerührt, bis die Biomasse vollständig suspendiert ist. Dabei wird die Temparatur der Suspension langsam auf 25° C erhöht.

Zur Suspension werden 22,5 l 0,2 mol/l NaOH, 1 % SDS unter Rühren mit ca. 80 RPM zugegeben und bei 25° C 5 Minuten inkubiert. Es werden 22,5 l Kaliumacetatpuffer (3 mol/l Kaliumacetatpuffer pH 5,5) unter Rühren zugefügt und die Temperatur der Biomasse möglichst schnell auf 4° C abgesenkt. Die Biomasse wird für 30 Minuten bei 26.000 x g und 4° C zentrifugiert. Der Überstand, der die Plasmid-DNA enthält, wird isoliert und über eine 5 µm Filterkerze klarfiltriert.

Im nächsten Schritt wird eine Chromatographie an Q-Sepharose durchgeführt. Der dekantierte Zentrifugenüberstand wird durch Zugabe von TE-Puffer (10 mmol/l Tris-HCl, 1 mmol/l EDTA pH 8,5 ± 0,2) auf 49 - 50 mS/cm Leitfähigkeit eingestellt und auf 5 ± 4° C abgekühlt. Die gesamte Chromatographie wird bei dieser Temperatur durchgeführt. Der Zentrifugationsüberstand wird auf die aquilibrierte Säule aufgezogen. Anschließend wird die Säule mit ca. 8 SV 10 mmol/l Tris-HCl, 1 mmol/l EDTA, 0,65 mol/l NaCl pH 8,5 ± 0,2 gewaschen.

Zur Elution wird an die Säule ein Gradient (5 SV Puffer A (10 mmol/l Tris-HCl, 1 mmol/l EDTA, 0,65 mmol/l NaCl pH 8,0 ± 2), 5 SV Puffer B (10 mmol/l Tris-HCl, 1 mmol/l EDTA, 0,85 mol/l NaCl pH 8,0 ± 0,2)) angelegt und das Eluat bei einer Flußrate von 5 bis 8 SV/h fraktioniert, die Detektion erfolgt bei 254 nm. Der Vorpeak (Verunreinigungen) wird vom Hauptpeak (Plasmid-DNA) abgetrennt, in dem der Hauptpeak ab ansteigender Flanke in einem separaten Gefäß aufgefangen wird.

Anschließend wird eine Chromatographie an Hydroxylapatit (HA Ceramic) bei 5 ± 4° C durchgeführt.

Äquilibrierungspuffer: 0,1 mol/l Kaliumphosphat, 6 mol/l Harnstoff pH 7, 0 ± 0,2.

Waschpuffer 1: 0,15 mol/l Kaliumphosphat, 6 mol/l Harnstoff pH 7,0 ± 0,2.

Waschpuffer 2: 0,02 mol/l Kaliumphosphatpuffer pH 7,0 ± 0,2.

Elutionspuffer: 0,5 mol/l Kaliumphosphat pH 7,0 ± 0,2.

Die Detektion erfolgt bei 254 nm mit einer UV-Detektor/Schreibereinheit. Als Eichlösung wird eine 1 % Produktlösung (Plasmid-DNA), vermessen mit einem kalibrierten Photometer verwendet.

Der Q-Sepharose-Pool wird auf eine Endkonzentration von 1,1 mmol/l Calciumchlorid gebracht und auf die äquilibrierte Säule aufgezogen.

Dann wird die Säule bei einer Flußrate von 5-8 SV/h nacheinander gewaschen mit:
1. 0,1 mol/l Kaliumphosphat, 6 mol/l Harnstoff pH 7,0 ± 0,2, bis am Detektor keine Absorption mehr erkennbar ist.
2. 2-4 SV, 0,15 mol/l Kaliumphosphat, 6 mol/l Harnstoff pH 7,0 ± 0,2.
3. 5 SV, 0,02 mol/l Kaliumphosphat pH 7,0 ± 0,2.

Im Anschluß an die Waschschritte wird mit 0,5 mol/l Kaliumphosphatpuffer pH 7,0 ± 0,1 eluiert. Der Peak wird gesammelt und als Plasmid-DNA-Ausgangslösung in die CFF eingesetzt.

### 1.2 Cross-Flow-Filtration

Die Plasmid-DNA-Ausgangslösung hat eine Plasmid-DNA-Konzentration von ca. 200 µg/ml und ein Volumen von ca. 3750 ml. Die CFF wird mit einer Retentatdurchflußrate von 100-200 l/h·m², einem Transmembrandruck von ca. 0,8 bar und einem Überströmdruck von ca. 1,2 bar durchgeführt. Mit Hilfe der CFF wird das Volumen auf ca. 50 ml konzentriert und das Retentat anschließend gegen TE-Puffer ( 10 mmol/l Tris-HCl, 1 mmol/l EDTA, pH 8,0) fließend diafiltriert, bis die Werte für pH und Leitfähigkeit von Retentat und TE-Puffer übereinstimmen. Nach Beendigung des Diafiltrationsvorganges wird das Retentat durch Verdünnung mit Diafiltrationspuffer auf eine Plasmid-DNA-Konzentration von 1 mg/ml eingestellt. Eine Probe der erstellten Plasmid-DNA-Lösung wird entnommen und diese wie unter Punkt 3.1 beschrieben analysiert.

### 2. Messung der Endotoxinabreicherung nach CFF

Eine Plasmid-DNA-Lösung mit einem Volumen von 100 ml wird mit 1000 EU der E-Toxate® Endotoxinstandardlösung auf 10 EU/ml aufgestockt und darauf als Ausgangslösung im Experiment eingesetzt. Die Lösung wird mit TE-Puffer auf 1000 ml verdünnt und dann mit Hilfe der CFF wieder auf das Ausgangsvolumen von 100 ml konzentriert (Konzentrat 1). Der Verdünnungs- und Konzentrierungsschritt wird viermal hintereinander wiederholt. Nach jedem Konzentrierungsschritt wird aus dem jeweiligen Konzentrat eine Probe entnommen (Proben: Konzentrat 2, 3, 4, 5) und die Endotoxinkonzentration der Probe mit dem Limulus-Amöbozyten-Lysat Verfahren analysiert.

### 3. Ergebnisse

### 3.1 CFF der Plasmid-DNA-Lösung

Die Plasmid-DNA-Lösung läßt sich mit Hilfe der CFF problemlos konzentrieren und diafiltrieren. Die Ergebnisse der Untersuchung sind in nachfolgender Tabelle zusammengefaßt.

| Parameter | PLASMID-DNA-Ausgangslösung (HA-Pool) | PLASMID-DNA nach CFF | Diafiltrationspuffer (TE): 10 mmol/l Tris/HCl, 1 mmol/l EDTA, pH 8.0 |
|---|---|---|---|
| Volumen (ml) | 3750 | 505 | - |
| OD260/280 | 1.89 | 1.90 | - |
| Leitfähigkeit (mS/cm) | 26.5 | 1.11 | 1.11 |
| pH | 6.99 | 7.93 | 7.98 |
| Ausbeute (mg) | 763 | 666 | - |

Ein Aliquot der Plasmid-DNA nach erfolgter CFF wird in verschiedenen Konzentrationen auf ein Agarosegel aufgetragen. Das dargestellte Agarosegel zeigt in den Spuren 1 und 10 den DNA Längenstandard Nr. II (Fragmentgrößen:125, 564, 2027, 2322, 4361, 6557, 9416, 23130 bp) und in den Spuren 2 und 9 DNA Längenstandard Nr. III (Fragmentgrößen: 125, 564, 831, 647, 1375, 1584, 1904, 2027, 3530, 4268, 4973, 5148, 21226 bp). Als Vergleichsplasmid ist in Spur 3 pBR322 (4162bp) aufgetragen, das nach konventioneller Cäsiumchloridgradientenmethode aufgereinigt wurde. Es ist bekannt, daß nach dieser Methode gereinigte Plasmid-DNA im wesentlichen Plasmid-DNA enthält, die der Konformation "Covalently Closed Circle" (dominante "Supercoiled-Bande") entspricht. In den Spuren 4, 5 und 6 ist die nach dem erfindungsgemäßen Verfahren aufgereinigte Plasmid-DNA (pCMV-CAT) in unterschiedlichen Mengen aufgetragen. Die erfindungsgemäß gereinigte Plasmid-DNA zeigt wie auch die Vergleichsplasmid-DNA (Spur 3) im wesentlichen eine dominante Bande. Diese Plasmid-DNA-Bande entspricht der Konformation "Covalently Closed Circle" (dominante "Supercoiled-Bande"). Dies zeigt, daß die erfindungsgemäß gewonnene Plasmid-DNA nicht geschädigt wird und in ihrer ursprünglichen Konformation erhalten bleibt. Somit kann ausgeschlossen werden, daß die Plasmid-DNA während der CFF fragmentiert oder in eine unerwünschte Plasmid-DNA-Konformation überführt wird.

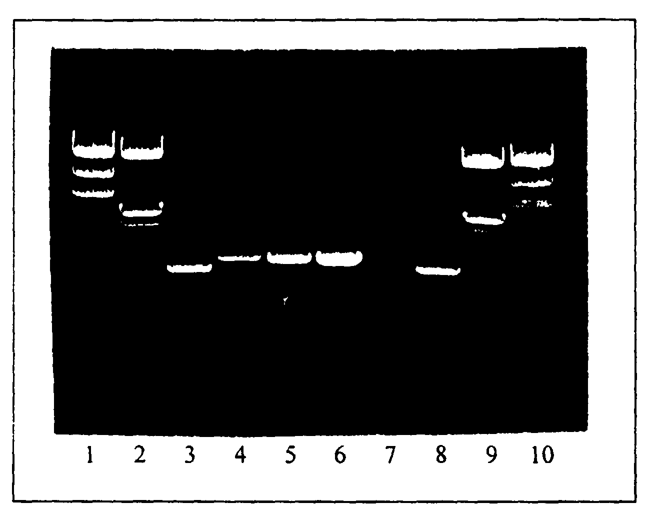

### Legende: 1%iges Agarosegel

- Spur 1:: DNA-Längenstandard II (Boehringer Mannheim GmbH, Kat.Nr. 236250)
- Spur 2:: DNA-Längenstandard III (Boehringer Mannheim GmbH, Kat.Nr. 528552)
- Spur 3:: pBR322 (0,4 µg (Boehringer Mannheim GmbH , Kat.Nr. 481238)
- Spur 4:: pCMV-CAT nach CFF, 0.19 µg (Bulk-Wirkstofflösung)
- Spur 5:: pCMV-CAT nach CFF, 0.45 µg (Bulk-Wirkstofflösung)
- Spur 6:: pCMV-CAT nach CFF, 0.71 µg (Bulk-Wirkstofflösung)
- Spur 7:: TE-Puffer
- Spur 8:: pBR322 (0,4 µg (Boeheringer Mannheim GmbH Kat.Nr. 481238)
- Spur 9:: DNA-Längenstandard III (Boehringer Mannheim GmbH, Kat.Nr. 528552)
- Spur 10:: DNA-Längenstandard II (Boehringer Mannheim GmbH, Kat.Nr. 236250)

### 3.2 Endotoxinabreicherung durch CFF

Die folgende Tabelle zeigt, daß die Endotoxine bereits nach dem ersten Konzentrierungsschritt weitgehend abgereinigt sind. Die weitere CFF reduziert die Endotoxinkonzentration bis zur Nachweisgrenze der Testmethode.

| Probe | Retentatvolumen [ml] | Gemessene Endotoxinkonzentration im Retentat [EU/ml] |
|---|---|---|
| Ausgangslösung | 100 | 6-12 |
| Konzentrat 1 | 100 | 0,06-0,60 |
| Konzentrat 2 | 100 | 0,06-0,60 |
| Konzentrat 3 | 100 | 0,06-0,60 |
| Konzentrat 4 | 100 | 0,06-0,60 |
| Konzentrat 5 | 100 | < 0,06 |
| Diafiltrationspuffer | - | < 0,06 |

## Patentansprüche

1. Verfahren zur Reinigung oder/und zur Konzentrierung von Nucleinsäuren in einer Lösung,
**dadurch gekennzeichnet,**
daß die Nucleinsäure enthaltende Lösung tangential an einer oder mehreren semipermeablen Membranen vorbeigeleitet wird, so daß die Nucleinsäure-Moleküle von den Membranen zurückgehalten werden und Substanzen mit einem geringeren Molekulargewicht durch die Membranen durchtreten können, wobei eine gereinigte oder/und konzentrierte Nucleinsäure-Lösung erhalten wird.

2. Verfahren nach Anspruch 1,
**dadurch gekennzeichnet,**
daß die Nucleinsäure Plasmid-DNA ist.

3. Verfahren nach Anspruch 1 oder 2,
**dadurch gekennzeichnet,**
daß die Nucleinsäure eine Größe von ≥ 150 Basenpaaren hat.

4. Verfahren nach einem der vorhergehenden Ansprüche,
**dadurch gekennzeichnet,**
daß das Verfahren kontinuierlich durchgeführt wird.

5. Verfahren nach einem der vorhergehenden Ansprüche,
**dadurch gekennzeichnet,**
daß eine Lösung mit einem Volumen von 1 - 10.000 l aufgearbeitet wird.

6. Verfahren nach einem der vorhergehenden Ansprüche,
**dadurch gekennzeichnet,**
daß eine Lösung mit einem Volumen von 1 - 100 l aufgearbeitet wird.

7. Verfahren nach einem der vorhergehenden Ansprüche,
**dadurch gekennzeichnet,**
daß die Lösung unter Druck an der/den Membran(en) vorbeigeleitet wird, wobei der Überströmdruck größer als der Transmembrandruck ist.

8. Verfahren nach Anspruch 7,
**dadurch gekennzeichnet,**
daß unter einem Transmembrandruck von 0,2 - 3,0 bar gearbeitet wird.

9. Verfahren nach einem der vorhergehenden Ansprüche,
**dadurch gekennzeichnet,**
daß mit einer Retentatfließrate von 100 - 4.000 l/h·m² gearbeitet wird.

10. Verfahren zur Abtrennung von Endotoxinen aus einer Nucleinsäure-Präparation,
**dadurch gekennzeichnet,**
daß die Nucleinsäure enthaltende Präparation tangential an einer oder mehreren semipermeablen Membranen vorbeigeleitet wird, so daß die Nucleinsäure-Moleküle von den Membranen zurückgehalten werden und Substanzen mit einem geringeren Molekulargewicht durch die Membranen durchtreten können oder/und von der Membran adsorbiert werden, wobei eine im wesentlichen endotoxinfreie Nucleinsäure-Lösung erhalten wird.

11. Verfahren nach einem der Ansprüche 1 - 10,
**dadurch gekennzeichnet,**
daß die Membran(en) eine Ausschlußgröße von 1 - 1000 kD hat/haben.

12. Verfahren nach einem der Ansprüche 1 - 10
**dadurch gekennzeichnet,**
daß die Membran(en) eine Ausschlußgröße von 10 - 100 kD hat/haben.

13. Verfahren nach einem der Ansprüch 10 - 12,
**dadurch gekennzeichnet,**
daß das Verfahren kontinuierlich durchgeführt wird.

14. Verfahren nach einem der Ansprüche 10 - 13,
**dadurch gekennzeichnet,**
daß eine Präparation mit einem Volumen von 1 - 10.000 l, vorzugsweise 1 - 100 l aufgearbeitet wird.

15. Verfahren nach einem der Ansprüche 10 - 14,
**dadurch gekennzeichnet,**
daß der Endotoxinabreicherungsfaktor, Endotoxingehalt der Präparation vor der Cross-Flow-Filtration (CFF) zu Endotoxingehalt der Lösung nach der CFF mindestens 10 : 1 beträgt.

16. Verfahren nach einem der Ansprüche 10 - 14,
**dadurch gekennzeichnet,**
daß der Endotoxinabreicherungsfaktor mindestens 200 : 1 beträgt.

17. Verfahren nach einem der Ansprüche 10 - 16,
**dadurch gekennzeichnet,**
daß der Endotoxingehalt der Lösung nach der Cross-Flow-Filtration < 0,1 EU/mg Nucleinsäure aufweist.

18. Verfahren nach einem der vorhergehenden Ansprüche,
**dadurch gekennzeichnet,**
daß die erhaltene Nucleinsäure im wesentlichen keine Strangbrüche aufweist.

19. Verwendung einer Cross-Flow-Filtrationsanlage zur Reinigung oder/und zur Konzentrierung von Nucleinsäuren in einer Lösung.

20. Verwendung einer Cross-Flow-Filtrationsanlage zur Abtrennung von Endotoxinen aus einer Nucleinsäure-Präparation.

21. Verwendung der nach einem der vorhergehenden Ansprüche gereinigten oder/und konzentrierten Nucleinsäuren zum Klonieren, zur Tranformation, zur Transfektion, zur Mikroinjektion in Zellen, zur Verwendung bei Verfahren der Gentherapie oder/und zur Polymerase-Ketten-Reaktion (PCR).
